(19) **Europäisches Patentamt** / **European Patent Office** / **Office européen des brevets**

(11) **EP 3 200 770 B1**

(12) # EUROPEAN PATENT SPECIFICATION

(45) Date of publication and mention
of the grant of the patent:
**02.09.2020 Bulletin 2020/36**

(21) Application number: **15787314.2**

(22) Date of filing: **29.09.2015**

(51) Int Cl.:
*A61K 9/127* (2006.01)     *A61K 36/81* (2006.01)
*A61K 9/20* (2006.01)     *A61K 9/48* (2006.01)
*A61P 9/10* (2006.01)

(86) International application number:
**PCT/IN2015/050124**

(87) International publication number:
**WO 2016/051424 (07.04.2016 Gazette 2016/14)**

(54) **A FORMULATION USEFUL FOR DELIVERY OF NEURO PROTECTING AGENT**

FORMULIERUNG ZUR VERABREICHUNG EINES NEURO-SCHUTZMITTELS

FORMULATION UTILE POUR L'ADMINISTRATION D'AGENT NEUROPROTECTEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.09.2014 IN 2773DE2014**

(43) Date of publication of application:
**09.08.2017 Bulletin 2017/32**

(73) Proprietor: **Council of Scientific and Industrial
Research
New Delhi 110001 (IN)**

(72) Inventors:
• **DWIVEDI, Anil Kumar**
  **Lucknow 226001 (IN)**
• **AHMAD, Hafsa**
  **Lucknow 226001 (IN)**
• **KHANDELWAL, Kiran**
  **Lucknow 226001 (IN)**
• **SANGWAN, Rajender Singh**
  **Lucknow 226015 (IN)**
• **SANGWAN, Neelam Singh**
  **Lucknow 226015 (IN)**
• **GAYEN, Jiaur Rahaman**
  **Lucknow 226001 (IN)**
• **., Sarika**
  **Lucknow 226001 (IN)**
• **BHADUARIA, Smrati**
  **Lucknow 226001 (IN)**
• **GAUR, Suresh Pratap Singh**
  **Lucknow 226001 (IN)**

• **BHOSALE, Vivek Vidyadhar**
  **Lucknow 226001 (IN)**
• **RATH, Srikanta Kumar**
  **Lucknow 226001 (IN)**
• **SHARMA, Sharad**
  **Lucknow 226001 (IN)**
• **SHUKLA, Rakesh**
  **Lucknow 226001 (IN)**

(74) Representative: **Heinze, Ekkehard
Meissner Bolte Patentanwälte
Rechtsanwälte Partnerschaft mbB
Widenmayerstraße 47
80538 München (DE)**

(56) References cited:
**WO-A1-01/03715          WO-A1-96/12472
WO-A1-2011/077100          WO-A2-2007/014334
US-A1- 2003 055 103**

• **AHMAD HAFSA ET AL: "Stability indicating
studies on NMITLI 118RT+ (standardized extract
of withania somnifera dunal).",
PHARMACOGNOSY MAGAZINE , 24 July 2014
(2014-07-24), pages 1-7, XP002752972, Retrieved
from the Internet:
URL:http://www.phcog.com/article.asp?issn=
0973-1296;year=2014;volume=10;issue=39;spa
ge=227;epage=233;aulast=Ahmad [retrieved on
2016-01-07]**

- CHAUDHARY GEETA ET AL: "Evaluation of Withania somnifera in a middle cerebral artery occlusion model of stroke in rats.", CLINICAL AND EXPERIMENTAL PHARMACOLOGY & PHYSIOLOGY 2003 MAY-JUN, vol. 30, no. 5-6, May 2003 (2003-05), pages 399-404, XP002752973, ISSN: 0305-1870

- AHMAD H1 ET AL: "Neuro-protective potential of a vesicular system of a standardized extract of a new chemotype of Withania somnifera Dunal (NMITLI118RT+) against cerebral stroke in rats", DRUG DELIVERY, ACADEMIC PRESS, ORLANDO, FL, US, 2 June 2015 (2015-06-02), pages 1-12, XP009187853, ISSN: 1071-7544, DOI: 10.3109/10717544.2015.1041579

## Description

### Field of the Invention

The present invention relates to a formulation useful for delivery of neuro protecting agent. It also demonstrates and discloses a novel delivery system for an extract of a new chemotype of *Withania sonmifera* (NMITLI118RT+) for neuro-protection against cerebral stroke (signifies concentrated aqueous ethanolic extract of roots of NMITLI-118, a new chemotype of *Withania somnifera,* a variety of Ashwagandha, which is unique in having high root yield, possessing a uniform crop canopy and uniform composition with respect to its secondary metabolites-withanolides. It is phytochemically characterized by particular abundance of two withanolides i.e. withanone and/or withanolide A, specimens of which are kept in the herbarium of CSIR-CIMAP, Lucknow, India) (Chaurasiya ND, Sangwan RS, Mishra L N, Tuli R, Sangwan N S, Metabolic clustering of a core collection of Indian ginseng Withania somnifera Dunal through DNA, Isoenzyme, polypeptide and withanolide profile diversity-Fitoterapia 2009, 80 (8) 496-505) for neuro-protection against cerebral stroke. Broadly, the invention discloses potent and prudent therapeutic potential of a new formulation using standardized fraction NMITLI118RT+ of a new variety of *Withania somnifera* (Ashwagandha) for brain function restoration and protection from neurological failures. Particularly the present invention further relates to the process for the preparation of the said formulation containing Standardized NMITLI118RT+ extract, its pharmaceutical compositions, for the management of cerebral stroke. The present disclosure also relates to a method of treatment of cerebral stroke and comprising of administering the formulation in a predetermined doses and period.

### Backgrounds of the invention:

Interaction between the active constituent and excipients can alter stability and bioavailability of drugs, thereby, affecting its safety and/or efficacy. Hence the careful choice of the excipients is imperative for formulation of an effective dosage form and the pharmaceutical development of solid dosage forms should imply a previous preformulation study of the drug and excipients. A number of experimental techniques (i.e., DSC, FT-IR spectroscopy, X-ray powder diffraction, Scanning Electron Microscopy, High Performance Liquid Chromatography (HPLC), etc.) have been used to investigate the interaction between drug and excipients.

Analytical studies were attempted on NMITLI118RT+ to generate data beneficial for formulation development. The fingerprinting pattern was recorded and Withanolide A contents were estimated in its different batches. On the basis of the results of stability studies and behavior of NMITLI118RT+ under stress conditions the following storage conditions are recommended: it should be protected from heat, moisture, aerial oxidation and acidic or alkaline pH. For formulation development pH 7 is the most suitable pH. Excipient studies were undertaken as a preliminary forerunner for formulation development and a range of excipients including lubricants, glidants, binders, polymers etc were chosen under the study. Maximum drug content and minimum interaction was achieved with ethyl cellulose while minimum drug content and maximum interaction was achieved with hydroxy propyl cellulose. The data generated hereby gives useful leads for further studies on NMITLI118RT+ formulations.

In order to assess and evaluate the toxicity profile of the test substance, NMITLI118RT+ determination of its adverse effects, if any, was done by performing a single dose toxicity study. The test protocol for this purpose was designed to obtain information on possible health hazards likely to arise in the test species from a single exposure of test substance by oral route. The animals were observed for fourteen days after single exposure to NMITLI118RT+ and various parameters indicative of systemic toxic effects were examined and evaluate. The study was intended to provide an estimate of the dose which was likely to be free of toxic effects on single exposure through oral route.

The results indicated that NMITLI118RT+ is a safe compound for drug development.

The various approaches in liposomal delivery systems can be beneficial in the effective administration of the active constituent and targeting. Liposomes are used as a drug carrier for such a therapeutic strategy as they have the ability to pass through the intercellular space between vascular endothelial cells and accumulate in the tissue owing to the enhanced permeability and retention (EPR) effect; and increasing vascular permeability is a strategy of choice in the treatment of many diseases like cerebral stroke. With this scientific intent, two formulations were designed: a blend and a novel liposomal drug delivery system. A blend (fill material) for solid dosage forms was formulated by liquid-solid compacting technique (overcoming the hygroscopic nature of NMITLI118RT+) with the aim that it could be used in comparison of biological activity present in the liposomal drug delivery system of NMITLI118RT+. Several attempts were made to prepare the required formulations most of them were found to be lacking in one or the other parameter. Ultimately we reach by selecting specific GRAS (generally regarded as safe) materials with their specific ratios and the method of preparation to get final formulations. In these formulations we prevent the interaction and degradation of the marker compounds to get best biological activity. Results were suggestive of the fact that the fill material approximated the anti-stroke activity of NMITLI118RT+; while the liposomal system exhibited significantly enhanced activity over the blend as well as NMITLI118RT+; additionally in the liposomal system the dose was successfully reduced to one-tenth of NMITLI118RT+.

**Prior Art:**

**Stroke**

Stroke is a major cause of morbidity and disability in industrialized countries and the overall costs of stroke care will account for 6.2% of the total burden of illness by 2020. (Demaerschalk, B.M.; Hwang, H.M.; Leung, G. US cost burden of ischemic stroke: A systematic, literature review. Am. J. Manag. Care 2010, 16, 525-533.) A stroke occurs when one of the arteries to the brain is either blocked or bursts and part of the brain does not get the blood it needs. A transient ischemic attack (TIA) occurs when the blood supply to the brain is blocked for a short time.

Ischemic stroke occurs when an artery in the brain is blocked. Embolic stroke and thrombotic stroke are the two types of ischemic stroke. In an embolic stroke, a blood clot or plaque fragment forms, usually in the heart or the large arteries leading to the brain, and then moves through the arteries to the brain. In the brain, the clot blocks a blood vessel and leads to a stroke. A thrombotic stroke is a blood clot that forms inside an artery that supplies blood to the brain. The clot interrupts blood flow and causes a stroke.

A hemorrhagic stroke happens when a blood vessel in the brain bursts and spills blood into or around the brain. The intracerebral hemorrhagic stroke is caused when a burst blood vessel bleeds into brain tissue. In another type of stroke, a blood vessel bursts near the surface of the brain and blood leaks in between the brain and the skull. This blood may cause nearby arteries to spasm, and that reduces blood flow to the brain and causes a stroke. (National Stroke Association, Explaining stroke, pg 1-18, http://www.stroke.org/site/DocServer/ExplainingStroke_web.pdf?docID=3321) In ischemic stroke, representing about 80% of all strokes, decreased or absent circulating blood deprives neurons of necessary substrates. The effects of ischemia are fairly rapid because the brain does not store glucose, the chief energy substrate and is incapable of anaerobic metabolism.

Non-traumatic intracerebral hemorrhage represents approximately 10% to 15% of all strokes. The pathophysiological changes that occur in response to stoke might be free radical production, excitotoxicity, disruption of sodium and calcium influx, enzymatic changes, stimulation of the inflammatory processes, endothelin release, activation of platelets and leukocytes, delayed coagulation and endothelial dysfunction. All these pathophysiological reactions contribute to the brain injury following onset of stroke. (Therapeutic potential of herbal drugs in cerebral ischemia.Y. K. Gupta, Seema Briyal, Anil Gulati. Indian J Physiol Pharmacol 2010; 54 (2): 99-122).

Ischemia is defined as diminution of cerebral blood flow (CBF) to a critical threshold that propagates brain damage involving the entire brain or a selective region. Cerebral ischemia/reperfusion (I/R) injury is a secondary impairment occurring after recovery from cerebral stroke. The, anti-apoptotic agents are anticipated to be beneficial for treating or preventing the injury. (A Single injection of liposomal asialo-erythropoietin improves motor function deficit caused by cerebral ischemia/reperfusion Takayuki Ishii, Tomohiro Asai, Tatsuya Fukuta, Dai Oyama, Nodoka Yasuda, Yurika Agato, Kosuke Shimizu, Tetsuo Minamino, Naoto Oku. International Journal of Pharmaceutics 439 (2012) 269-274). Owing to the gross complexities associated with the pathophysiology of stroke possibly the drugs of different mechanisms of action may be more effective than an approach based on an individual candidate. A drug directed against a single molecular target may not be as effective at treating the neuronal cell death associated with stroke. Thus need for effective preventive therapy, and for early critical care in patients with stroke becomes imperative (Lapchak, P.A.; Schubert, D.R.; Maher, P.A. Delayed treatment with a novel neurotrophic compound reduces behavioral deficits in rabbit ischemic stroke. J. Neurochem. 2010, 116, 122-131).

The only FDA approved drug for stroke treatment is recombinant tissue plasminogen activator (r-tPA; alteplase) or prolyse (pro-urokinase) but with a narrow therapeutic time window of 3 hours. Recent clinical trials have failed to demonstrate unequivocal benefit using later time points in stroke patients. Hence efforts were made to study the preventive as well as the curative effect of extract and formulations of *Withania somnifera* on cerebral stroke, so that it could possibly be used as an alternative.

*Withania somnifera, Centella asiatica,* Shilajit, *Tinospora cordifolia* and *Convolvulus pluricaulis* have been evaluated as prophylactic treatment in the middle cerebral artery occlusion model of stroke in rats (Chaudhary G, Sharma U, Jagannathan NR,Gupta YK. Evaluation of Withania somnifera in a middle cerebral artery occlusion model of stroke in rats. Clin Exp Pharmacol Physiol. 2003; 30: 399-404). (Therapeutic potential of herbal drugs in cerebral ischemia.Y. K. Gupta, Seema Briyal, Anil Gulati. Indian J Physiol Pharmacol 2010; 54 (2): 99-122).

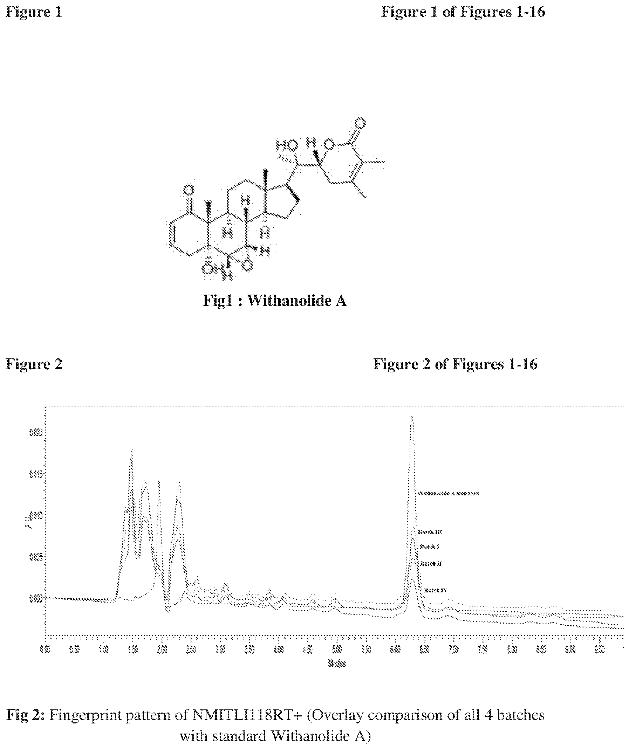

Out of the many species of genus Withania (Solanaceae) *Withania somnifera* Dunal also known as Ashwagandha, Indian ginseng or winder cherry is the most important plant of medicinal significance in the traditional Indian systems of medicine such as Ayurveda, Unani and Siddha for over 3000 years. Some important classical uses of the plant includes its use as an aphrodisiac and in liver tonic, anti-inflammatory bronchitis, asthma, ulcers, emaciation, insomnia and senile dementia. The significant medicinal potential of *W. somnifera* is mainly associated with the presence of unique classes of steroidal lactones called Withanolides. These are ergostane based moieties; triterpenoidal in origin and include pharmaceutically active molecules like Withanolide A (figure 1), Withaferin A, Withanolide D and Withanone etc. (Chaurasiya ND, Sangwan RS, Misra LN, Tuli R, Sangwan NS. Metabolic clustering of a core collection of Indian ginseng Withania somnifera Dunal through DNA, isoenzyme, polypeptide and withanolide profile diversity. Fitoterapia. 2009 Dec;80(8):496-505, Dhar RS, Verma V, Suri KA, Sangwan RS, Satti NK, Kumar A, et al. Phytochemical and genetic analysis in selected chemotypes of Withania somnifera. Phytochemistry. 2006 Oct;67(20):2269-76, Rajasekar S, Elango R. Estimation of alkaloid content of Ashwagandha (Withania somnifera) with HPLC Methods. J Exp Sci. 2011; 2(5):39-41).

Some of the clinically proven properties of *W. somnifera* in recent times happen to be cell cycle disruption and anti-angiogenic potential (Mathur R, Gupta SK, Singh N, Mathur S, Kochupillai V, Velpandian T. Evaluation of the effect of Withania somnifera root extracts on cell cycle and angiogenesis. J Ethnopharmacol. 2006, 105, 336-41). It has also been demonstrated to possess adaptogenic, anti-inflammatory, antioxidant, anti-platelet, antihypertensive, hypoglycemic and hypolipidemic effects which may contribute to its cardio-protective properties (Ojha SK, Arya DS. Withania somnifera Dunal (Ashwagandha): A promising remedy for cardiovascular diseases. World J Med Sci. 2009, 4, 156-58). Its anti-stress and anti-hypertensive effects have also been validated by Kushwaha et al 2012 (Kushwaha S, Betsy A, Chawla P. Effect of Ashwagandha (Withania somnifera) Root Powder Supplementation in Treatment of Hypertension. Ethno Med. 2012, 6, 111-15). Ashwagandha is also said to possess antitumor, anti-stress, immunomodulatory, hemopoetic, and rejuvenating properties. (Mishra LC, Singh BB, Dagenais S. Scientific basis for the therapeutic use of Withania somnifera (Ashwagandha): A review. 2000, 5, 334-46) Its anxiolytic and antidepressant potential has also been reported by Bhattacharya et al (Bhattacharya SK, Bhattacharya A, Sairam K, Ghosal S. Anxiolytic-antidepressant activity of Withania somnifera glycowithanolides: an experimental study. Phytomedicine. 2000, 7, 463-9). The potential immune-prophylactic efficacies of several root extracts of *W. somnifera* chemotypes NMITLI-101, NMITLI-118, NMITLI-128 against an infective filarial pathogen was also studied. (Kushwaha S, Soni VK, Singh PK, Bano N, Kumar A, Sangwan RS, et al. Withania somnifera chemotypes NMITLI 101R, NMITLI 118R, NMITLI 128R and withaferin A protect Mastomys coucha from Brugia malayi infection. Parasite Immunol. 2012, 34, 199-209). Many approaches for broad based chemical analysis have been used to identify targeted and non-targeted metabolites in roots and leaves of *W. somnifera* and to quantify them. 62 major and minor primary and secondary metabolites from leaves and 48 from roots were unambiguously identified. (Chaurasiya ND, Sangwan RS, Misra LN, Tuli R, Sangwan NS. Metabolic clustering of a core collection of Indian ginseng Withania somnifera Dunal through DNA, isoenzyme, polypeptide and withanolide profile diversity. Fitoterapia. 2009, 80, 496-505) Phytochemical analysis and its correlation to genetic factors has been dealt by Dhar et al (Dhar RS, Verma V, Suri KA, Sangwan RS, Satti NK, Kumar A, et al. Phytochemical and genetic analysis in selected chemotypes of Withania somnifera. Phytochemistry. 2006, 67, 2269-76) Quantitative HPLC methods for Withanolide D and Withaferin A have also been reported (Ganzera M, Choudhary MI, Khan IA. Quantitative HPLC analysis of withanolides in Withania somnifera. Fitoterapia. 2003, 74, 68-76).

The ethnopharmalogical properties of *Withania somnifera* Dunal (Solanaceae) include adaptogenic, anti-sedative and anti-convulsion activities, and then plant has been employed in the treatment of neurological disorders, geriatric debilities, arthritis and stress- and behaviour-related problems (Schliebs *et al.,* 1973; Ray and Gupta, 1994; Bhattacharya *et al*., 1997; Kulkarni *et al*., 1998; Dhuley, 2001).

Several chemotypes of Withania somnifera (WS) like NMITLI-101 (101R), NMITLI-118 (118R) and NMITLI-128 (128R) were developed by CSIR-CIMAP, Lucknow. Several biological properties were reported in these chemotypes. In recent times the different chemotypes of *Withania somnifera* demonstrated excellent immune-stimulatory properties. It is also worth mention that chemotypes, with several-fold variability in the content of individual withanolides have been reported in *W. somnifera*. (Withania somnifera chemotypes NMITLI 101R, NMITLI 118R, NMITLI 128R and withaferin A protect Mastomys coucha from Brugia malayi infection, S. Kushwaha, V. K. Soni, P. K. Singh, N. Bano, A. Kumar, R. S. Sangwan & S. Misra-Bhattacharya, Parasite Immunology, 2012, 34, 199-209)

WO 01/03715 A1 discloses a high purity Withania Somnifera extract in the form of a stable, free-flowing, light yellow-to-brown herbaceous powder, for producing an enhanced cognition effect for the user and to augment the learning facility in the geriatric population when taken in a dosage of 200-800 mg/day.

WO 2007/014334 A2 provides a method of treatment of various adaptogenic conditions such as stress in humans, comprising administering Withania somnifera plant extract.

Too many choices neither exist in the present scenario for effective treatment of stroke nor are satisfactory delivery systems of existing drug candidates available for the same. Hence it could be said that therapeutic strategies for the treatment of acute stroke have not yet been achieved, due to poor efficacy and to adverse side effects in clinical trial.

Therefore, an increase in drug efficacy with reduced side effects is highly desirable for achieving neuro-protection in stroke patients.

To overcome the complexities associated with drug delivery of anti-stroke agents, liposomal drug delivery system was thought of as an alternative in the treatment of cerebral stroke. Liposomes are spherical particles with one or multiple concentric membranes in which a fraction of the solvent can be encapsulated. Liposomes are constructed of polar lipids. Typical compositions include phosphatidylcholines and phosphatidylethanolamines, often containing negatively charged lipids, such as phosphatidylserine and phosphatidylinositol. In addition ceramides (such as sphingomyelin), and sterols (cholesterol, ergosterol, sitosterol, etc.) are also included. (Olga Gortzi, Stavros Lalas, Ioanna Chinou, John Tsakins, Reevaluation of bioactivity and antioxidant activity of Myrtus communis extract before and after encapsulation in liposomes. Eur Food Res Technol, 2007, Springer-Verlag, 1-8). In addition, polyethylene glycol-modified liposomes (PEGylated liposomes) possess a long circulating property in the bloodstream by avoiding interaction with opsonins and the cells of the mononuclear phagocytic system PEGylated liposomes have been used to increase drug stability, safety, and bioavailability in humans. Liposomal systems can anticipatedly have better pharmacological effect of the neuro-protectant. Liposome encapsulation of active principles enhances their bioavailability to the brain. Hence it could be said that liposomes offer a wide range of merits and could be thought of as a suitable delivery system for the administration of neuro-protectants in the treatment of cerebral stroke.

Several agents that find mention in the treatment of stroke and have been developed as liposomal systems can be briefed here: Liposome encapsulation of Cytidine-5 V-diphosphocholine also called CDP-choline or Citicoline (i.v.) provides more efficient delivery to the ischemic brain and thus increases brain uptake. Liposomes remain in circulation for more than 24 h thereby providing a sustained release of the drug. (CDP-choline liposomes provide significant reduction in infarction over free CDP-choline in stroke Rao Muralikrishna Adibhatla, J.F. Hatcher, K. Tureyen Brain Research 1058 (2005) 193-197).

Encapsulation of citicoline in liposomes increases its therapeutic effects in ischemia, as evident in a longitudinal MRI study of lesion volumes and edema in an animal model of stroke. (Serial MRI study of the enhanced therapeutic effects of liposome-encapsulated citicoline in cerebral ischemia Pedro Ramos-Cabrer, Jesus Agulla, Bárbara Argibay, Maria Pérez-Mato, Jose Catillo. International Journal of Pharmaceutics 405 (2011) 22 8-233.) Liposomes modified with the antiapoptotic protein asialoerythropoietin were found to significantly suppress cerebral cell death and improve motor functional deficits induced by I/R injury in transient middle cerebral artery occlusion (t-MCAO) rats by increasing the accumulation of the protein in the injured region. (Treatment of cerebral ischemia-reperfusion injury with PEGylated liposomes encapsulating FK506. Takayuki Ishii, Tomohiro Asai, Dai Oyama, Yurika Agato, Nodoka Yasuda, Tatsuya Fukuta, Kosuke Shimizu, Tetsuo Minamino, and Naoto Oku. The FASEB Journal 2 Vol. 27 April 2013 pg 1-9)

AEPO-modified PEG-ylated liposomes (Asialo-erythropoeitin) as a neuro-protective agent were developed and examined for their therapeutic efficacy in model rats with transient cerebral ischemia. Suggestively it was able to cause amelioration of cerebral ischemia-reperfusion injury (Takayuki Ishii, Tomohiro Asai, Dai Oyama, Tatsuya Fukuta, Nodoka Yasuda, Kosuke Shimizu, Tetsuo Minamino, Naoto Oku, Journal of controlled release, Vol 160 issue 130 may 2012, 81-87)

However none of the probable candidates have made it to the clinical trials due to shortcomings in dosing and side effects.

Liposomes encapsulating extracts possess new physicochemical characteristics and bioactivity which can enhance extract's original activity and thus with this intent the inventors designed novel delivery systems of NBMITLI118RT+ for effective treatment of cerebral stroke; which successfully reduced the dose to one-tenth of NMITLI 118RT+ extract and exhibited significant improvement against cerebral ischemic injury.

Choice of Phospholipids:
Phosphoglycerides, Sphingolipids, Phosphatidylcholine like Egg phosphatidylcholine, Soy lecithin, Phosphatidylethanolamine, Phosphatidylserine, Synthetic phospholipids like DOPC: Dioleoyl-phosphatidylcholine, DSPC: Distearoyl-phosphatidylcholine.
Choice of Sterols:
Cholesterol, Ergosterol, Sitosterol
Choice of Binder: Saccharides and their derivatives: Disaccharides: sucrose, lactose; polysaccharides and their derivatives: starches, cellulose or modified cellulose such as microcrystalline cellulose and cellulose ethers such as hydroxypropyl cellulose (HPC); Sugar alcohols such as xylitol, sorbitol etc Protein: gelatin, synthetic polymers: polyvinylpyrrolidone (PVP), polyethylene glycol (PEG)
Choice of disintegrating material:
Starch, explotab, crosspovidone
Choice of Carrier material:
Microcrystalline cellulose (MCC) such as pH101, and 200. Avicel® RTM 105, Avicel® pH 102 granular, microcrystalline cellulose (MCC) grade, avicel® pH 200 coarse granular MCC grade, experimental grade of granular amorphous cellulose, starch, lactose
Choice of Glidant:
Magnesium stearate, talc
Choice of Coating material:
Silica of various grades (Cab-O-Sil M5), Aerosil 200, Syloid 244FP, hydroxy propyl methyl cellulose (HPMC)
Choice of Solvents:
Ethanol, methanol, dichloromethane, distilled water.

**Objects of the present invention**

The main object of this invention is to provide a novel delivery system of NMITLI118RT+ for neuro-protection against cerebral stroke.

Further object of this invention is to deliver NMITLI 118RT+ in the form of a liposomal drug delivery system. Another object of this invention is to provide a pharmaceutically acceptable NMITLI118RT+ preparation without potentially toxic organic solvents.

Yet another object of this invention is to provide a formulation of NMITLI118RT+, which can target the drug to the brain for a longer duration.

Further object of this invention is to provide stable formulation, which can be used for the treatment of cerebral stroke.

Further object of this invention is to protect NMITLI118RT+ from moisture to control its hygroscopic nature so that the marker compounds may be protected.

**Summary of the invention:**

Accordingly, the present invention provides a formulation useful for delivery of neuro protecting agent which comprises said neuroprotective agent in a vehicle, the vehicle being a liposome, wherein said neuro protecting agent is root extract of *Withania somnifera* designated as NMITLI118RT+.

In an aspect not forming part of the invention the disclosure provides a formulation wherein the ingredients ratios in the formulation for capsules and tablets is root extract of new chemotype of *Withania somnifera* (NMITLI118RT+) (35-55%), a filler (5-20%), a binder (2-5%), a disintegrating material (2-5%), a glidant (0.1-2%), a coating material (0.1-5%), using a mixture of a diol and primary alcoholic as vehicle-carrier system for soublising NMITLI118RT+.

The binder is selected from disaccharides like sucrose, lactose, polysaccharides like starches, cellulose, modified cellulose (eg microcrystalline cellulose), cellulose ethers (eg hydroxypropyl cellulose), sugar alcohols such as xylitol, sorbitol, gelatin, synthetic polymers such as polyvinylpyrrolidone (PVP), polyethylene glycol (PEG), the disintegrating material may be selected from starch, explotab, crosspovidone, the carrier material may be selected from microcrystalline cellulose granular amorphous cellulose, starch, lactose, the glidant may be selected from magnesium stearate, talc, the coating material may be selected from silica of various grades, aerosil 200, syloid 244FP, and the solvents for preparation may be selected from propylene glycol, glycerol, ethanol, 2-propanol, n-propanol and thereof.

In an embodiment the invention provides a formulation wherein the lipid is selected from the group comprising of phosphatidylcholine (egg phosphatidylcholine, soy lecithin), phosphoglycerides, sphingolipids, phosphatidylethanolamine, phosphatidylserine, synthetic phospholipids like DOPC: dioleoyl-phosphatidylcholine, DSPC: distearoyl-phosphatidylcholine.

In an embodiment the invention provides a formulation wherein the sterol is selected from the group comprising of cholesterol, ergosterol, sitosterol, and thereof, and the solvents may be selected from ethanol, methanol, dichloromethane, distilled water.

In an embodiment the invention provides a formulation wherein the concentration of the root extract of the new chemotype of *Withania somnifera* (NMITLI118RT+) is 0.1-5.0 mg/mL.

In an embodiment the invention provides a method for preparing the formulation comprising the liposomal vehicle, comprising the steps :

a. Solubilising 5 mg of root extract of new chemotype of *Withania somnifera* (NMITLI118RT+) in 1 mL ethanol;
b. Adding 4 mL dichloromethane to the mixture obtained in step a;
c. Solubilising Soy lecithin (S) 40 mg and Cholesterol (C) 2 mg in the solvent mixture obtained in step b;
d. Evaporating the content obtained in step c to dryness on a rotary evaporator at 140 rpm and 50 °C for 1 hour to obtain a film;
e. Drying the film obtained in step d;
f. Rehydrating the film obtained in step e with 5 mL phosphate buffer (pH; 7.0) at 50°C for 1 hour to obtain a liposomal dispersion;
g. Homogenizing the liposomal dispersion obtained in step f to obtain the formulation.
h. Purification of the liposomal dispersion obtained in step g by lyophilization.

In an embodiment the invention provides a formulation useful for brain function restoration, protection from neurological failures, and cerebral stroke.

In an embodiment the invention provides a formulation wherein the concentration of liposome is 2-48 mg.

In another aspect of the present disclosure not forming part of the invention wherein the delivery system for capsules and tablets the binder is selected from disaccharides like sucrose, lactose, polysaccharides like starches, cellulose, modified cellulose (eg microcrystalline cellulose), cellulose ethers (eg hydroxypropyl cellulose), sugar alcohols such as xylitol, sorbitol, gelatin, synthetic polymers such as polyvinylpyrrolidone (PVP), polyethylene glycol (PEG), the disintegrating material may be selected from starch, explotab, crosspovidone, the carrier material may be selected from microcrystalline cellulose granular amorphous cellulose, starch, lactose, the glidant may be selected from magnesium stearate, talc, the coating material may be selected from silica of various grades, aerosil 200, syloid 244FP, and the solvents for preparation may be selected from propylene glycol, glycerol, ethanol, 2-propanol, n-propanol and thereof.

In another aspect of the present disclosure wherein the method of treatment of brain function restoration, protection from neurological failures and cerebral stroke by administering the formulation in predetermined doses and period.

## Detailed description of the invention

The following examples broadly illustrate the nature of this invention the manner in which it is to be performed without limiting the nature and scope of the invention.

## Example - 1 (not according to the invention)

**Extracts preparation of a new chemotype of *Withania somnifera* (NMITLI118RT+):** Freshly harvested roots (average ratio of FW:DW; 100: 27) of NMITLI118 were cleaned with distilled water, de-moistened on Whatman No. 1 filter paper with tissue paper and weighed. 3 Kg of the cleaned and de-moistened roots were subjected to size-reduction and powdered in liquid $N_2$. The root powder was extracted with two volumes (w/v, 2 L per Kg fresh weight) of 25 % ethanol (v/v) in water for 12 h at room temperature (25 to 30 °C). Extract was filtered through two layers of muslin cloth and the filtrate stored at 4°C. The residue was re-extracted for 12h with 1.5 volumes (w/v, 1.5 L per Kg fresh weight) of 25 % (v/v) ethanol in water and filtered. Filtrate was saved as above, and residue re-extracted for 12 h in one volume of 25% ethanol in water to get the extract (filtrate) as above. All the three filtrates were pooled and filtered through a Whatman No. 1 filter paper using a vacuum pump filtration system. The clarified filtrate was concentrated under reduced pressure using a rotary evaporator until the filtrate reached to slurry stage. The concentrated extract was lyophilized to dry powder stage, sealed and immediately stored at -80°C. Aliquots of the lyophilized powder were kept separately for TLC and HPLC analyses.

The average yield of extract was 62 g per Kg fresh weight of root.

## Example 2 (not according to the invention)

## Finger printing of the NMITLI118RT+

**HPLC- Chromatographic Conditions:** Waters HPLC (Milford, MA, USA) system used was equipped with a binary gradient pump (model 515, Waters), an auto sampler injector (Model 2707, Waters) and a diode array detector (Model 2998, Waters). Waters HPLC interface and Empower 2 software was utilized for data acquisition. HPLC resolution of Withanolide A was achieved on a RP-18e B Lichrosphere® column (250 X 4 mm, 5µm, Merck, Germany) at 30±3° C utilizing mobile phase consisting of acetonitrile: water (1:1); flow rate being 1.0 mL/min and injection volume remained 20 µL. Column effluent was monitored at 240 nm (PDA detection).

**Standard Withanolide A solution:** About 1 mg of Withanolide A was accurately weighed and dissolved in 100 µL dimethyl sulfoxide and subsequently volume was made up to 10 ml with methanol to achieve a concentration of 100 µg/mL. Further dilutions in the range 2 to 50 µg/mL were made from this stock by serial dilution method.

**Test Solution of NMITLI118RT+:** Test solutions of 1 mg/mL were prepared in methanol-water (1:1).

The method was validated as per ICH guide lines and validation parameters are given in table 1.

**Table 1:** Assay of Withanolide A (Validation Parameters)

| S.No. | Validation Parameters | Values |
|---|---|---|
| 1 | LOD | 0.5 µg/mL |
| 2 | LOQ | 2.0 µg/mL |
| 3 | Linearity range | 2 µg/mL -100 µg/mL |
| 4 | Regression equation | y = 27933x - 21605 |
| 5 | $R^2$ | $R^2$= 0.999 |

(continued)

| S.No. | Validation Parameters | Values |
|---|---|---|
| 6 | Recovery range | 97.50%-101.75% |

**Fingerprint profile of NMITLI118RT+:** 4 batches of NMITLI118RT+ (maintained under refrigeration) were analyzed for determination of their Withanolide A content and fingerprint pattern. The fingerprint profiles were recorded (figure2) and Withanolide A content (table 2) was estimated. The fingerprint pattern remained largely identical in all 4 batches, however variation in Withanolide A content was observed. A typical chromatogram for NMITLI118RT+ is shown in figure 2.

**Table 2:** Estimation of Withanolide A content in different batches of NMITLI118RT+

| Sample specifications | Withanolide A Conc. $\mu$g/mg |
|---|---|
| Batch-I | 4.250 |
| Batch-II | 2.211 |
| Batch III | 4.658 |
| Batch-IV | 1.219 |

**Example - 3 (not according to the invention)**

**Drug Excipient Studies:** Since the concerned drug here is an herbal extract an isothermal stress testing (IST) methodology (binary compatibility study) was designed which was analyzed by a stability indicating procedure by HPLC. Equimolar mixtures (100 mg each) of the NMITLI118RT+ extract and the chosen excipients were maintained at 40 °C $\pm$ 2°C for a period of 7 days in glass vials. A control sample containing only the extract was also kept under similar conditions. After 7 days all samples were withdrawn and 5 ml methanol-water (1:1) solution was added to each vial. These samples were then individually vortexed to ensure mixing and then filtered. The subsequent filtrate achieved of concentration 20 mg/ml was further used to achieve the final test samples of 1 mg/ml each. These were analyzed by HPLC for estimation of Withanolide A content and to observe any changes in fingerprint pattern or additional peaks.

All samples containing the chosen excipients and the control were analyzed; their Withanolide A content was estimated (table 3) and all observations recorded. The fingerprint profile remained largely similar in all samples; as many as 17 peaks could be detected in the control sample. An additional peak at retention time 6.9 mins was observed in control and in samples with ethyl cellulose, dextrose and PEG, whereas it remained absent in samples containing hydroxy propyl cellulose, lactose, talc, stearic acid, zinc stearate and eudagrit. Lowest numbers of peaks were seen in the chromatogram of sample containing HPC and the lowest amount of Withanolide A content was recorded for the same.

Order of Withanolide A Content amongst different samples:

Control > Ethyl cellulose > Starch> PEG 6000 > Dextrose > Zinc stearate > Eudagrit L 100 > Talc > Stearic acid > Magnesium stearate > Lactose> Hydroxy propyl cellulose

**Table 3: Estimation of Withanolide A content in NMITLI118RT+-excipient samples**

| Sample Specification | Withanolide A content ($\mu$g/mL) |
|---|---|
| Control | 4.530 |
| NMITLI118RT+ + Ethyl cellulose | 3.793 |
| NMITLI118RT+ + Starch | 3.112 |
| NMITLI118RT+ + PEG6000 | 2.854 |
| NMITLI118RT+ + Dextrose | 2.549 |
| NMITLI118RT+ + Zinc stearate | 2.542 |
| NMITLI118RT+ + Eudagrit L 100 | 2.375 |
| NMITLI118RT+ + Talc | 2.322 |
| NMITLI-118R + Stearic acid | 2.145 |

(continued)

| Sample Specification | Withanolide A content ($\mu$g/mL) |
|---|---|
| NMITLI118RT+ + Magnesium stearate | 2.092 |
| NMITLI118RT+ + Lactose | 1.979 |
| NMITLI118RT+ + Hydroxy propyl cellulose | 0.940 |

**Example 4**

NMITLI118RT+ was dissolved in glycerol; which was incorporated in a blend comprising of magnesium stearate, starch and ethyl cellulose. The composition is as follows:

| Materials | Parts per gram (for a 600 mg blend) |
|---|---|
| NMITLI118RT+ | 430mg |
| Magnesium stearate | 10 mg |
| Starch | 60 mg |
| Ethyl Cellulose | 100 mg |

**Inference: The blend obtained showed clumping and was non-uniform in consistency.**

**Example 5 (not according to the invention)**

NMITLI118RT+ dissolved in glycerol-ethanol mixture which was incorporated in a blend comprising of magnesium stearate, starch and ethyl cellulose. The composition is as follows:

| Materials | Parts per gram (for a 600 mg blend) |
|---|---|
| NMITLI118RT+ | 430mg |
| Magnesium stearate | 5 mg |
| Starch | 30 mg |
| Ethyl Cellulose | 165 mg |

**Inference: The blend obtained was better, however with residual tackiness and not completely free flowing.**

**Example 6 (not according to the invention)**

The vehicle was changed to propylene glycol with incorporation of aerosil in the blend formula along with lactose, magnesium stearate and starch. A blend was obtained by liquid-solid compacting. The composition is as follows:

| Materials | Parts per gram |
|---|---|
| NMITLI118RT+ | 430mg |
| Lactose | 421mg |
| Magnesium stearate | 2.5 mg |
| Starch | 30mg |
| Aerosil | 116.5 mg |

**Inference: Blend properties improved, however flowability was a problem.**

**Example 7 (not according to the invention)**

Introduction of vehicle-carrier system: propylene glycol-ethanol to dissolve NMITLI118RT+, which was incorporated in a blend comprising lactose, magnesium stearate, starch and aerosil by compacting technique. The composition is as follows:

| Materials | Parts per gram |
|---|---|
| NMITLI118R T+ | 430mg |
| Lactose | 421mg |
| Magnesium stearate | 1mg |
| Starch | 30mg |
| Aerosil | 118 mg |

**Inference: Tackiness reduced flowability better but complete free flowing stage yet to be achieved.**

**Example 8 (not according to the invention)**

A blend for fill material to prepare solid dosage form was formulated for NMITLI118RT+ by solid liquid compacting technique utilizing Propylene glycol- ethanol as vehicle and Aerosil as coating material. A completely free flowing blend of uniform consistency marked with absence of any tackiness was obtained by reversing the ratio of lactose and aerosil as in the previous example and the composition per gram of fill material is as follows:

| Materials | Parts per gram |
|---|---|
| NMITLI118RT+ | 430mg |
| Lactose | 118mg |
| Magnesium stearate | 1mg |
| Starch | 30mg |
| Aerosil | 421 mg |

**Inference: Free flowing uniform blend without any greasiness or tackiness; ready to be taken up for further characterization and biological activity.**

**Example 9 (not according to the invention)**

**Characterization of Blend obtained in Example 9**

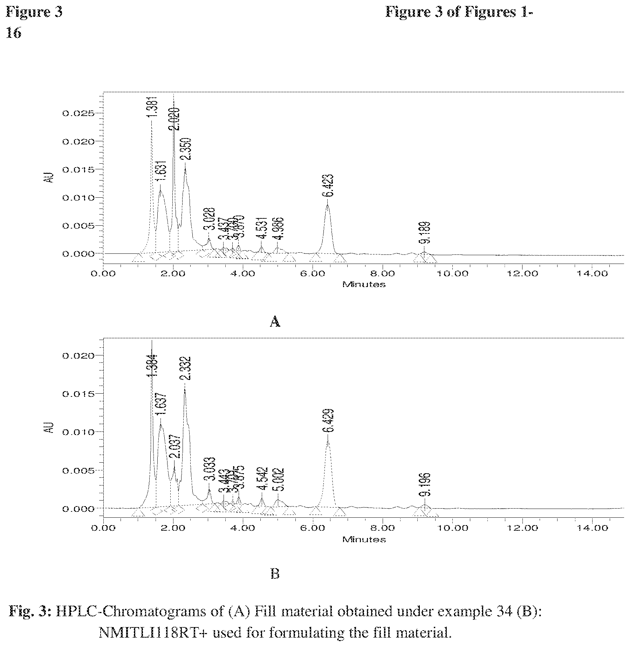

**Fill Material Analysis by HPLC:** 23.25mg fill material was dissolved in 10 ml methanol and allowed to stand for 2 hours. The solution was subsequently vortexed; filtered and run for estimation of Withanolide A content by HPLC which was found to be 5.192 $\mu$g/mg which closely approximated the NMITLI118RT+ used for fill material preparation in Withanolide A content and fingerprint pattern. (Fig 3)
**% of Material in sample:**

$$\text{Wt of equivalent material} = 2.325 \times 430/1000 = 0.99975 \text{ mg/ml.}$$

$$\% \text{ of material in sample} = \text{Peak area of sample} \times \text{wt of std.} \times 100/\text{Peak area of std.} \times \text{wt of sample}$$
$$= 123435 \times 1 \times 100/123295 \times 0.99975 = 100.11\%$$

**Example -10 (not according to the invention)**

**Preparation of blank liposomal delivery system 1 (L1)**

Egg phosphatidylcholine (EPC); 48 mg and Cholesterol (C); 12 mg were solublised in 10 mL dichloromethane-methanol mixture (4:1). The contents were introduced in a round bottom flask and evaporated to dryness on a rotary evaporator at 120 rpm and 50 °C; to obtain a film. The film was allowed to dry for 30 mins and to prevent separation of phospholipid membrane by hydrolysis; it was rehydrated with 100 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.
**Inference: Ridging and clumping of material, absence of film formation and size and pdi values were not within limits.**

**Example - 11 (not according to the invention)**

**Preparation of blank liposomal delivery system 2 (L2)**

Soy lecithin (S); 48 mg and Cholesterol (C); 12 mg were solublised in 5 mL dichloromethane- methanol mixture (4:1). The contents were introduced in a round bottom flask and evaporated to dryness on a rotary evaporator at 130 rpm and 50 °C; to obtain a film. The film was allowed to dry for 30 mins and to prevent separation of phospholipid membrane by hydrolysis; it was rehydrated with 10 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.
**Inference: Ridging and clumping of material, absence of film formation and pdi values were not within limits.**

**Example - 12 (not according to the invention)**

**Preparation of blank liposomal delivery system 3 (L3)**

Soy lecithin (S); 25 mg and Cholesterol (C); 20 mg were solublised in 5 mL dichloromethane- methanol mixture (4:1). The contents were introduced in a round bottom flask and evaporated to dryness on a rotary evaporator at 130 rpm and 60 °C; to obtain a film. The film was allowed to dry for 30 mins and to prevent separation of phospholipid membrane by hydrolysis; it was rehydrated with 10 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.
**Inference: Film failed to form and size and pdi values were higher than required.**

**Example - 13 (not according to the invention)**

**Preparation of blank liposomal delivery system 4 (L4)**

Soy lecithin (S); 40 mg and Cholesterol (C); 5 mg were solublised in 5 mL dichloromethane- methanol mixture (4:1). The contents were introduced in a round bottom flask and evaporated to dryness on a rotary evaporator at 130 rpm and 50 °C; to obtain a film. The film was allowed to dry for 30 mins and to prevent separation of phospholipid membrane by hydrolysis; it was rehydrated with 10 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.
**Inference: Film formed with partial ridging and size and pdi values were not within limits.**

**Example - 14 (not according to the invention)**

**Preparation of blank liposomal delivery system 5 (L5)**

Soy lecithin (S); 40 mg and Cholesterol (C); 5 mg were solublised in 5 mL dichloromethane- methanol mixture (4:1). The contents were introduced in a round bottom flask and evaporated to dryness on a rotary evaporator at 150 rpm and 50 °C; to obtain a film. The film was allowed to dry for 30 mins and to prevent separation of phospholipid membrane by hydrolysis; it was rehydrated with 10 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.

Inference: Film formed with partial ridging and size and pdi values were not within limits.

**Example - 15 (not according to the invention)**

**Preparation of blank liposomal delivery system 6 (L6)**

Soy lecithin (S); 40 mg and Cholesterol (C); 2 mg were solublised in 5 mL dichloromethane- ethanol mixture (4:1). The contents were introduced in a round bottom flask and evaporated to dryness on a rotary evaporator at 140 rpm and 50 °C; to obtain a film. The film was allowed to dry for 30 mins and to prevent separation of phospholipid membrane by hydrolysis; it was rehydrated with 10 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.

Inference: Best observed film with size and pdi values well within range.

**Example - 16**

**Preparation of NMITLI118RT+ loaded liposomal delivery system 1 (F1)**

NMITLI118RT+; 2mg, Soy lecithin (S); 40 mg and Cholesterol (C); 2 mg were solublised in 5 mL dichloromethane- methanol mixture (4:1). The contents were introduced in a round bottom flask and evaporated to dryness on a rotary evaporator at 140 rpm and 50 °C; to obtain a film. The film was allowed to dry for 30 mins and to prevent separation of phospholipid membrane by hydrolysis; it was rehydrated with 10 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.

Inference: Clumping was largely observed, size and pdi were not in good agreement.

**Example - 17**

**Preparation of NMITLI118RT+ loaded liposomal delivery system 2 (F2)**

NMITLI118RT+; 2mg was solublised in 1 mL methanol and 4 mL dichloromethane was added to it, subsequently Soy lecithin (S); 40 mg and Cholesterol (C); 2 mg were solublised in this solvent mixture. The contents were introduced in a round bottom flask and evaporated to dryness on a rotary evaporator at 140 rpm and 50 °C; to obtain a film. The film was allowed to dry for 30 mins and to prevent separation of phospholipid membrane by hydrolysis; it was rehydrated with 10 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.

Inference: Film formed but with breakage, size and pdi were within limits.

**Example - 18**

**Preparation of NMITLI118RT+ loaded liposomal delivery system 3 (F3)**

NMITLI118RT+; 10 mg was solublised in 1 mL ethanol and 4 mL dichloromethane was added to it, subsequently Soy lecithin (S); 40 mg and Cholesterol (C); 2 mg were solublised in this solvent mixture. The contents were introduced in a round bottom flask and evaporated to dryness on a rotary evaporator at 140 rpm and 50 °C; to obtain a film. The film was allowed to dry for 30 mins and to prevent separation of phospholipid membrane by hydrolysis; it was rehydrated with 15 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer.

Inference: A good film was formed; size and pdi were in limits.

**Example - 19**

**Preparation of NMITLI118RT+ loaded liposomal delivery system 4 (F4; formulation chosen for further biological evaluation)**

NMITLI118RT+; 5 mg was solublised in 1 mL ethanol and 4 mL dichloromethane was added to it, subsequently Soy lecithin (S); 40 mg and Cholesterol (C); 2 mg were solublised in this solvent mixture. The contents were introduced in a round bottom flask and evaporated to dryness on a rotary evaporator at 140 rpm and 50 °C; to obtain a film. The film was allowed to dry for 30 mins and to prevent separation of phospholipid membrane by hydrolysis; it was rehydrated with 5 mL phosphate buffer (pH; 7.0) at 50°C. It was maintained in hydration for 1 hour. The liposomal dispersion was then homogenized with a probe sonicator and filtered through a 0.45 μ syringe filter and evaluated for size and pdi on Malvern zeta sizer and stored until further use. **Inference: Best observed film with size and pdi in good agreement.**
Optimization and development of liposomal delivery systems (Examples 22-34) as described in Table 4.

**Table 4:** Development of blank and drug loaded formulations and their particle size measurements

| Label | Composition (mg) | | Conditions | | Size Z Avr.: (nm) | PDI |
|---|---|---|---|---|---|---|
| | Lipid1 1 | Lipid 2 | rpm | Temperature (°C) | | |
| L1 | 48 | 12 | 120 | 50 | 156.70 ±20.0 | 0.534 ±0.06 |
| L2 | 48 | 12 | 130 | 50 | 116.00 ±13.0 | 0.581 ±0.03 |
| L3 | 25 | 20 | 130 | 60 | 330.60 ±22.0 | 0.734 ±0.17 |
| L4 | 40 | 5 | 130 | 50 | 112.40 ±18.0 | 0.738 ±0.09 |
| L5 | 40 | 5 | 150 | 50 | 133.96 ±12.0 | 0.335 ±0.06 |
| L6 | 40 | 2 | 140 | 50 | 134.34±09.0 | 0.264 ±0.04 |
| F4 | 40 | 2 | 140 | 50 | 142.60±09.0 | 0.268±0.04 |
| Values are expressed **mean ± SD**; (n=3) | | | | | | |

**Example - 20**

**Characterization of formulation**

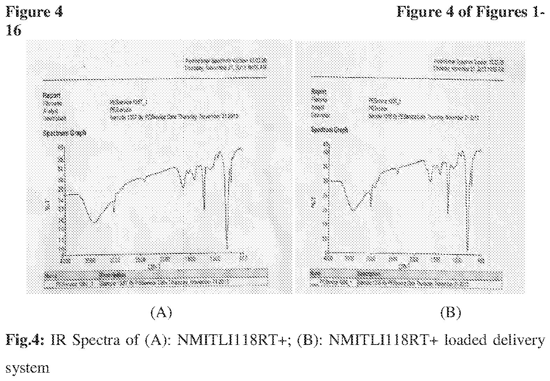

**Determination of mean particle size and poly dispersity index and zeta potential** -The mean particle size and particle size distribution of blank formulations as well as NMITLI118RT+ loaded preparation was evaluated by a laser diffraction particle size analyzer Zeta-sizer Nano ZS model (Malvern Instruments). The sample was dispersed in TDW and sonicated at 10% amplitude for 30 second. Optical properties of the sample were defined as follows: refractive index 1.47 and absorption 1.00. Each sample was measured in triplicate. The Z average (d. nm) was estimated to be 142.6± 0.09 and the poly-dispersity index was found to be 0.268 ± 0.04 for the final formulation. Zeta potential was also determined by laser Doppler anemometry using a Malvern Zeta-sizer, which was found to be -31.20 ± 1.0 mV.
**Determination of Encapsulation efficiency-** Unloaded 118RT+ was recovered from a 2 mL volume of liposome solution by passing the liposome solution through a 0.45 μm filter, and degrading the solution with 10 mL of ethanol. The ethanol was evaporated in a rotary evaporator and 118R was then re-dissolved in 2 mL of ethanol. The concentration of Withanolide A (major marker in 118R) was determined using HPLC, and the following Equation was used to calculate the encapsulation efficiency of 118R in liposomes on the basis of Withanolide A content. EE = $C_e$/ $C_i$ * 100 where $C_e$ = Concentration of encapsulated drug and $C_i$ = Initial concentration of drug
The encapsulation efficiency was found to be 94.603% ±2%
**IR Spectroscopy-** Infrared (IR) spectra were measured on carefully dried samples embedded in KBr pellets. IR spectra of both 118R as well as the 118R loaded liposomal preparation were obtained. Both were similar; however an additional peak (Peak 3; % T: 27.83; 2928.49 cm$^{-1}$) corresponding to the altered composition due to lipid entrapment was observed. (Fig 4)
**Stability of developed formulation (F4)** - The final formulation was prepared in 3 batches and assessed over a 3 week study period for measurements on particle size and zeta potential. Results exhibited that the prepared formulation was stable over the study period, as there were negligible changes in the particle size and zeta potential measurements. (Table 5)

**Table 5:** Physical stability of developed system as evident from particle size measurement

| Batches of F4 | Particle size Z Avr. (d. nm) | | Zeta Potential (mV) | |
|---|---|---|---|---|
| | Initial | After 3 weeks | Initial | After 3 weeks |
| 1 | 141.25±09.46 | 140.51±07.48 | -31.20 ± 1.00 | -31.08 ± 1.15 |
| 2 | 139.48±11.12 | 138.73±12.47 | -28.63 ± 1.25 | -28.49 ± 1.08 |
| 3 | 142.73±07.21 | 142.28±09.26 | -31.53 ± 2.36 | -31.39 ± 2.12 |
| Values are expressed **mean ± SD;** (n=3) | | | | |

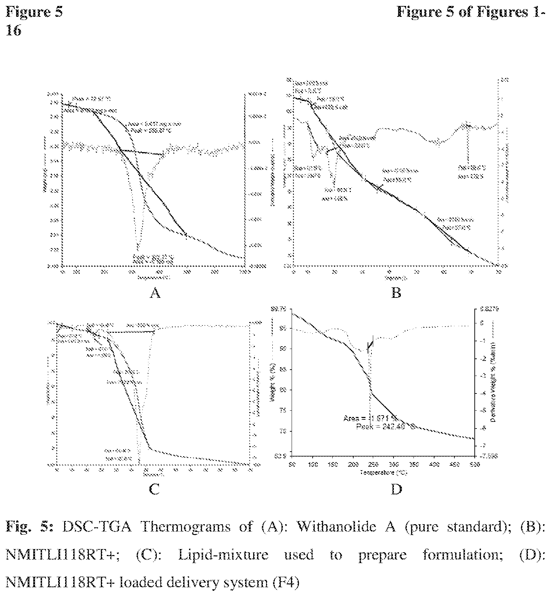

**DSC-TGA Analysis-** Thermo-gravimetric analysis curves of the samples of pure Withanolide A, NMITLI-118RT+, lipid mixture used for the preparation of formulation and final 118R loaded delivery systems were collected with a thermo analyzer within a temperature range of 50-700° C with increasing temperature at the rate of 10° C/min. (Fig 5)

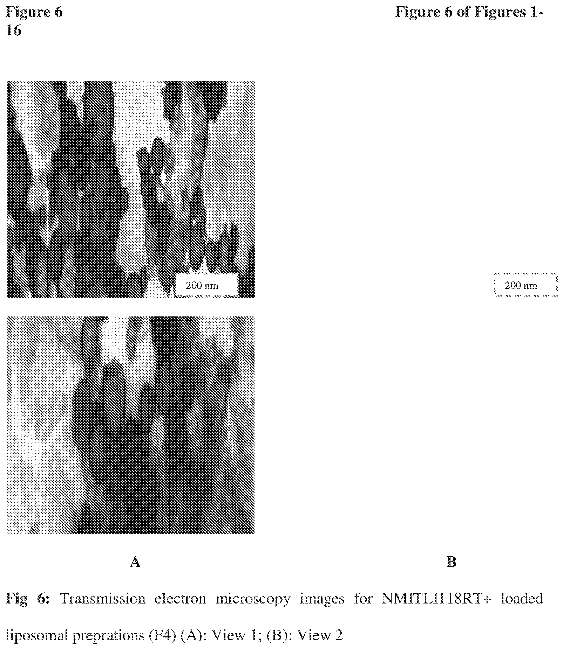

**TEM Studies-** The morphology of the drug loaded 118R liposomal delivery system was examined by transmission electron microscopy (TEM). TEM (TEM; 400T TEM, Philips, New Brunswick, Canada) studies were carried out using phosphotungstic acid as a negative stain. TEM was carried out to determine the surface characteristics of the optimized formulations in aqueous medium using a 3 mM forman (0.5% plastic powder in amyl acetate)-coated copper grid (300 mesh) at 60 kV using negative staining by 2% phosphotungstic acid at various magnifications. Results revealed morphologically spherical and globular particles around the Z Avr. being 200 nm. (Fig 6)

**Example 21**

**Biological activity (Anti Stroke)**

**Animals:**

Male Sprague-Dawley rats (240-260 gm) were procured from the National Laboratory Animal Centre of CDRI, Lucknow, India and experiments were performed after necessary approval of the Institute Animal Ethical Committee. Rats were allowed free access to food and water

**Animal Grouping**

Male Sprague-Dawley rats were divided in the following groups (n=8) for study:

| Sham | This group includes fresh male SD rats where all surgical procedure was carried out as in MCAo model except insertion of nylon filament. | |
|---|---|---|
| Control | All surgical procedure was carried out, middle cerebral artery was blocked by insertion of filament, and only vehicle (gum accacia) was given by oral route. | |
| NMITLI118RT+ Treated (50 mg/kg p.o.) | a | Treatment was given 1 hr prior to the ischemic insult |
| | b | Treatment was given after 6 hr of I/R injury. |
| Liposomal formulation of NMITLI118RT+ treated (5 mg/kg p.o.) | a | Treatment was given 1 hr prior to the ischemic insult |
| | b | Treatment was given after 6 hr of I/R injury. |
| Fill material of NMITLI118RT+ treated (50 mg/kg p.o.) | a | Treatment was given 1 hr prior to the ischemic insult |
| | b | Treatment was given after 6 hr of I/R injury. |

**Experimental protocol:** The middle cerebral artery occlusion (MCAO) model is the most preferred model to study cerebral stroke, because majority of human ischemic strokes results from occlusion of the middle cerebral artery, and thus MCAo model simulates the clinical situation.

**Surgical Procedure for induction of cerebral ischemia by MCAo:**

Chloral hydrate (300mg/kg i.p.) was used to anaesthetize Sprague Dawley rats (260±20g) and then rats were placed in a supine position over a preheated operation table to maintain the body temperature at 37°C±0.5°C. Through a midline incision in the neck region the left common carotid artery (CCA) was exposed. The neck muscles were separated further to expose external carotid artery (ECA) and internal carotid artery (ICA). A silk suture knot was tied on the ICA loosely close to the bifurcation region. A (3-0) nylon monofilament suture (Ethicon, Johnsons & Johnsons Ltd, Mumbai) was introduced into the ECA lumen through a nick given in the external carotid artery. The intraluminal suture blocked the origin of the MCA, occluding all sources of the blood flow from the ICA, anterior cerebral artery (ACA) and posterior cerebral artery (PCA). The suture was pulled back after 2hrs of ischemia to reestablish the cerebral blood flow.

After the designated time points of ischemia/reperfusion rats were examined for neurologic deficit, re-anaesthetized with ether and about 3.5ml of blood was taken out retro-orbitally to determine the MDA and GSH levels in the blood followed by TTC staining to assess the brain damage and infarction volume.

**Assessment of I/R injury**

    **a) Neurobehavioral Assessment:** The neurological deficit was scored on a 10-point scale following the method of Longa et al., 1989 and Bederson et al., 1986.

    **b) Malondialdehyde (MDA) estimation in blood:** The amount of malondialdehyde (MDA) formed as a by-product of lipid peroxidation was quantitated by reaction with thiobarbituric acid (TBA) in blood serum and has been used as an index of lipid peroxidation. The pink colored MDA-TBA complex formed at low pH and high temperature was measured at 532 nm by spectrophotometer.

    **c) GSH estimation in blood:** Glutathione was estimated in blood by the 5, 5"dithiobis-(2-nitrobenzoic acid) (DTNB)-glutathione reductase coupled assay essentially as described earlier. The yellow color edchromogen formed by reaction of GSH with DTNB was read at 412 nm with spectrophotometer.

    **d) Histological study by TTC Staining:** Brain infarction following cerebral ischemia/reperfusion injury was visualized with 0.5% 2,3,5 triphenyltetrazoliumchloride (TTC) staining. Viable tissue stains deep red while the infarct remains unstained. Hence, TTC staining is a suitable procedure for detection of infarcts in brain slices. Rat brain was perfused transcardially with normal saline and carefully isolated in chilled condition immediately. Cerebellum was removed and the brain was kept in refrigerator at -20°C. Frozen brain was sliced anterio-posteriorly into uniform sections of 2 mm thickness.

**Results:**

**Assessment of cerebral injury:**

**a) Effect of treatments on MDA level**

MDA is a by-product of lipid peroxidation. The MDA levels were measured post 24 hour of ischemic/reperfusion injury from blood serum obtained from rat orbital plexus. A significant increase in blood MDA levels (62.83%) was observed in vehicle treated ischemic rats as compared to sham rats.

**Pre treatment**

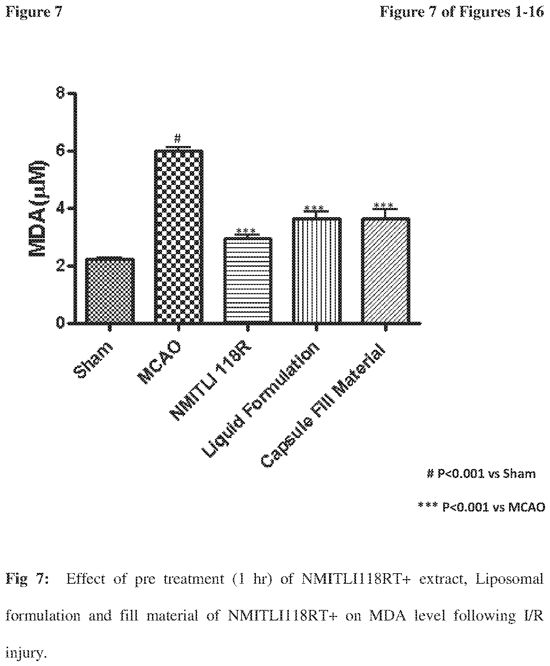

A decrease of about 50.66% in the MDA level was observed in rats treated with NMITLI118RT+. Whereas, the Liposomal formulation of NMITLI118RT+ at a dose of 5 mg/kg p.o. offered a decrease in the MDA level by 42.50% followed by a decrease by 39.16% by fill material of NMITLI118RT+ at a dose of 50 mg/kg p.o. (Fig 7)

**Post treatment**

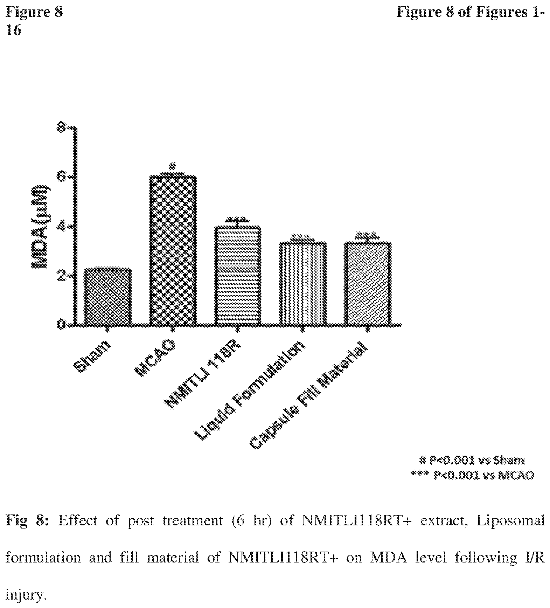

On the other side in a different set of experiment, when the rats were post treated with Liposomal formulation of NMITLI118RT+ at a dose of 5 mg/kg p.o. attenuated the MDA level by 44.83% and an equally significant effect(44.33%) was seen in fill material of NMITLI118RT+ rats at a dose of 50 mg/kg p.o.Whereas, only a reduction of 24.83% was observed in rats treated with NMITLI118RT+ extract at a dose of 50 mg/kg p.o. (Fig. 8) These studies indicated that the NMITLI 118RT+ extract and its formulation has reduced ischemic damage leading to reduction in MDA content in treated rats.

**b) Effect of treatments on GSH level**

GSH levels are markers of oxidative stress. The GSH levels were measured post 24 hour of I/R injury in the plasma of ischemic animals. A significant decline in GSH levels generally means an increase in oxidative stress, and was measured post 2/24hr of ischemic injury. A significant decline in blood GSH levels (59.67%) was observed in ischemic rats as compared to sham rats.

**Pre treatment**

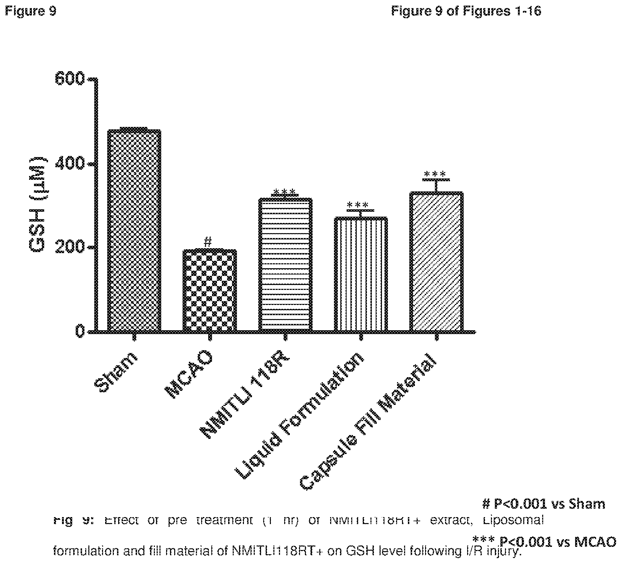

The fill material of NMITLI 118RT+ at a dose of 50 mg/kg p.o. proved to be effective in increasing the depleted levels of GSH by about 70.98%. Rats treated with the extract of NMITLI118RT+ showed an increase in GSH level by about 63.29% at a similar dose. A significant increase of 49.38% was observed in the rats treated with Liposomal formulation of NMITLI118RT+ at a dose of 5 mg/kg p.o. (Fig 9)

**Post treatment**

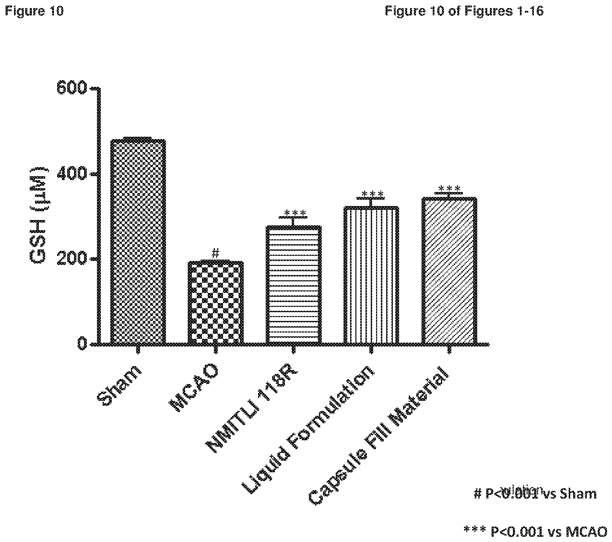

An overwhelming response was noticed in the rats treated with NMITLI118RT+ extract and its formulations. Fill material showed an increase by 76.93%, followed by an increase by 66.89% and 64.55% offered by Liposomal formulation and NMITLI118RT+ respectively. (Fig 10)

Thus the extract and its formulation seem to be a potent antioxidant and counteract oxidative stress due to ischemic insult.

**c) Effect of treatments on Neurological Deficit score**

Neurological deficit was analyzed on the basis of neurological scores obtained after 24 hr of reperfusion in all experimental groups. The neurological deficit score in treated group was significantly reduced when compared with control group at 2/24 hr of I/R injury.

**Pre treatment**

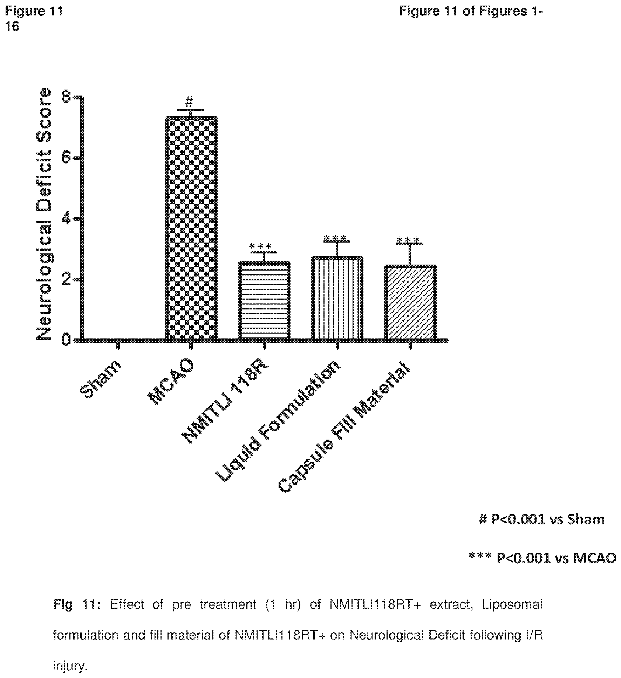

It was found that there was a significant reduction in the neurological score by 69.98% in rats pretreated with Liposomal formulation, 67.25% by fill material and 64.93% by NMITLI 118RT+. (Fig 11)

**Post treatment**

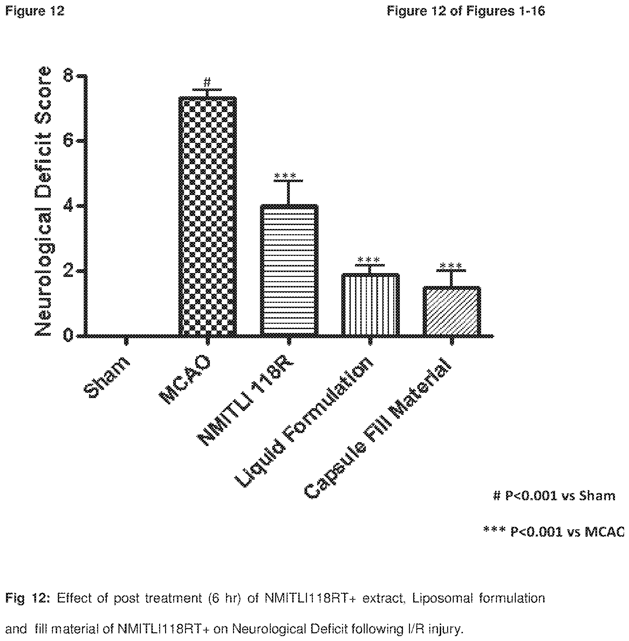

During this study, it was noticed that fill material when post treated had the ability to reduce the Neurological Deficit score by 79.53% as compared to Liposomal formulation which also had the same protective effect in reducing the neurological impairment by 75.44%. But only a decrease by 37.65% was shown by the rats treated with NMITLI118RT+ extract. (Fig 12)

These studies indicate that treatment with the extract and its formulation was significantly effective in preventing brain damage.

**d) Effect of treatments on cerebral infarct**

TTC staining is widely used to assess the cerebral damage resulting from ischemic insult. The rats used for the assessment of ND were sacrificed and thick brain sections (~2mm) were made and stained with TTC to assess the infarct area. The TTC unstained area representing infarct was measured and presented as percentage reduction in infract area. Control group produced an infarct size of almost 70.74% in ipsilateral side of control rat brain, mostly comprising of striatal and some of the cortical area as well.

**Pre treatment**

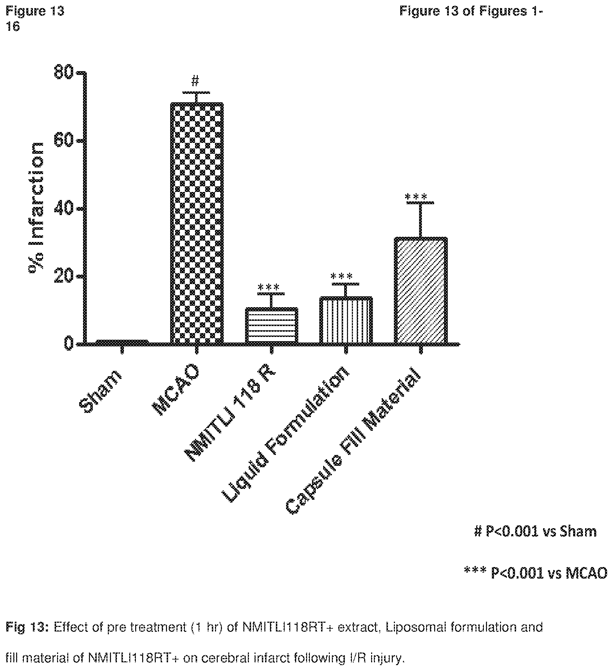

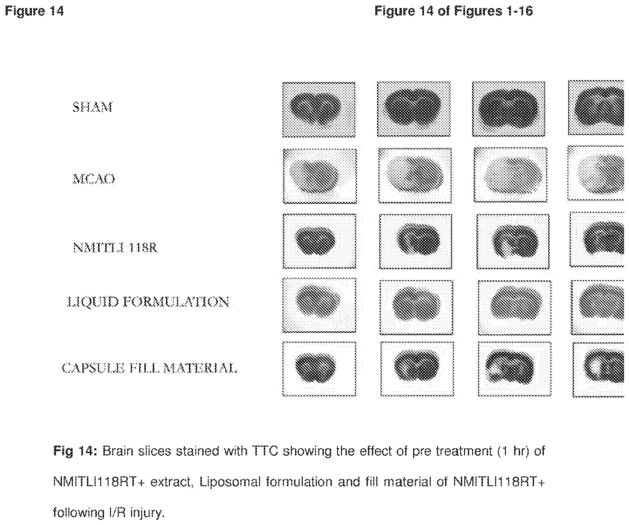

The pre treatment of rats with NMITLI118RT+ and its Liposomal formulation showed a good reduction in the cerebral infarction by 85% each. Whereas, a reduction by 55.71% was observed in rats treated with fill material of NMITLI118RT+ (Fig 13, Fig 14)

## Post treatment

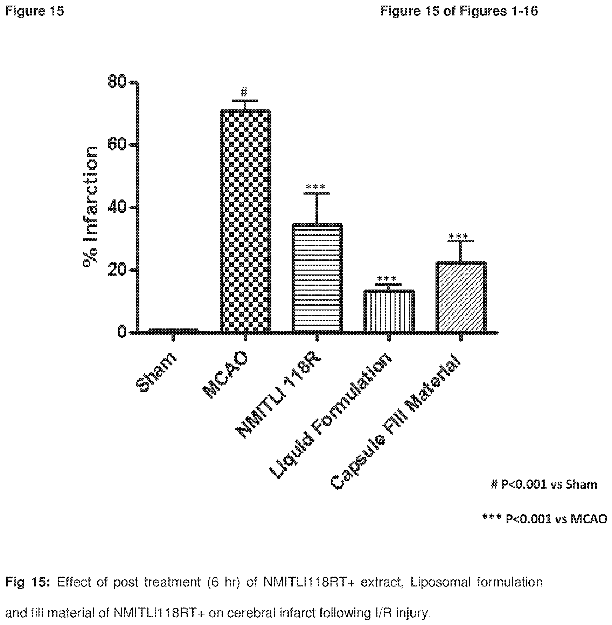

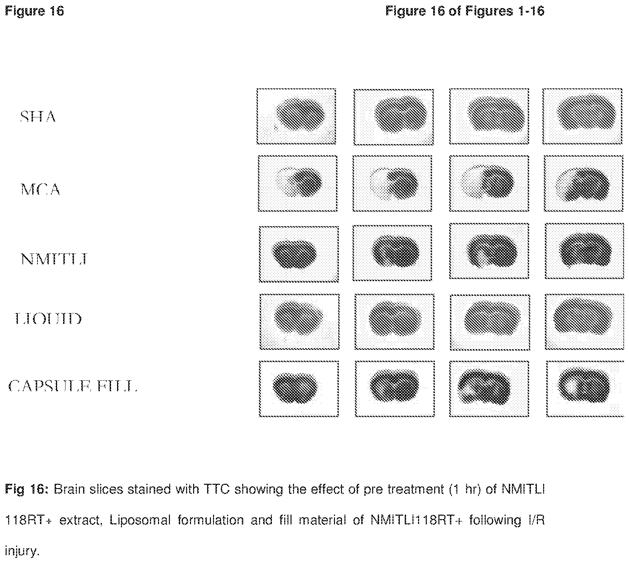

The effect of post treatment with Liquid formulation was similar to that of the effect shown by pre treatment on cerebral infarct. It was able to reduce the cerebral damage by 81.41 %. The Capsule fill material helped to reduce the brain infarct by 68.33% followed by a reduction of 44.52% offered by the NMITLI118R T+ extract. (Fig 15, Fig 16) Therefore, these results clearly demonstrate that the NMITLI118RT+ extract and its formulation has a potent neuroprotective activity in cerebral ischemia.

## Advantages of the present invention

Based on the toxicity data NMITLI118RT+ is a safe material for the drug development. It is a highly hygroscopic material and overcoming its hygroscopicity (which is responsible for degradation of one of the marker compound Withanolide A) was a major problem as far as formulation development was concerned especially when aiming at solid dosage forms (capsules/ tablets/ blends).
Here two approaches could possibly be adopted

1. Opting for a novel drug delivery system (liposomal preparation in the present case), which is targeted and require less amount of dose.
2. Overcoming its hygroscopic nature and developing a suitable solid dosage form (a blend was designed by compacting).

Several experiments were performed to reach the final set of formulations as claimed in this patent.

- These formulations are novel and overcome most of the shortcomings of the candidate drug material.
- The present formulations contain the dose equal to or less than the original NMITLI118RT+ therefore it is safer.
- This study also provides significant insights about the neuro-protective effect of novel formulations of NMITLI118RT+ in cerebral injury.
- Liposomal formulation was found to be equally effective at 1/10th dose of NMITLI118RT+.
- A particular advantage of the current invention is that it provides a simple and inexpensive system to facilitate the administration of medicaments.
- In many embodiments, this drug delivery system enhances the stability and bioavailability of pharmaceutically active agents, synergistically effective for desired pharmacological action.
- According to one aspect of this invention, the pharmaceutical formulation is administered through various routes including, but not limited to, oral, vaginal, rectal, intravenous, intradermal, intramuscular, subcutaneous, and intraperitoneal.
- In another aspect of the invention, a pharmaceutical formulation can be delivered in suspension and/or liquid suspension form.

Results clearly demonstrated that the NMITLI118RT+ extract and its formulations have a potent neuro-protective activity in cerebral ischemia.

## Claims

1.    A formulation useful for delivery of neuro-protecting agent, which comprises said neuro-protective agent in a vehicle, the vehicle being a liposome , wherein said neuro-protecting agent is root extract of *Withania somnifera* designated as NMITLI118RT+.

2.    The formulation as claimed in claim 1, wherein the liposome comprises lipid selected from the group consisting of phosphatidylcholine (egg phosphatidylcholine, soy lecithin), phosphoglycerides, sphingolipids, phosphatidylethanolamine, phosphatidylserine, synthetic phospholipids like DOPC:dioleoyl-phosphatidylcholine, DSPC: distearoyl-phosphatidylcholine and a sterol is selected from the group comprising of cholesterol, ergosterol, sitosterol, and thereof, and the solvents may be selected from ethanol, methanol, dichloromethane, distilled water.

3.    A method for preparing the formulation as claimed in claim 1, comprising the steps:

a. solubilising 5 mg of root extract of new chemotype of *Withania somnifera* (NMITLI118RT+) in 1 mL ethanol;
b. adding 4 mL dichloromethane to the mixture obtained in step a;
c. solubilising Soy lecithin (S) 40 mg and Cholesterol (C) 2 mg in the solvent mixture obtained in step (b);
d. evaporating the content obtained in step (c) to dryness on a rotary evaporator at 140rpm and 50°C for 1 hour to obtain a film;
e. drying the film obtained in step d;
f. rehydrating the film obtained in step (e) with 5 mL phosphate buffer (pH; 7.0) at 50°C for 1 hour to obtain a liposomal dispersion;
g. homogenizing the liposomal dispersion obtained in step (f) to obtain the formulation; and
h. purification of the liposomal dispersion obtained in step (g) by lyophilization.

4. The formulation as claimed in claim 1 useful for brain function restoration, protection from neurological failures, and cerebral stroke.

5. The formulation as claimed in claim 1-4 wherein the concentration of the root extract of the new chemotype of *Withania somnifera* (NMITLI118RT+) is 0.1-5.0 mg/mL.

**Patentansprüche**

1. Formulierung, die zur Abgabe von neuroprotektivem Mittel verwendbar ist, die das neuroprotektive Mittel in einem Vehikel umfasst, wobei das Vehikel ein Liposom ist, wobei das neuroprotektive Mittel Wurzelextrakt von *Withania somnifera*, bezeichnet als NMITLI118RT+, ist.

2. Formulierung, wie in Anspruch 1 beansprucht, wobei das Liposom Lipid, ausgewählt aus der Gruppe, bestehend aus Phosphatidylcholin (Eiphosphatidylcholin, Sojalecithin), Phosphoglyceriden, Sphingolipiden, Phosphatidylethanolamin, Phosphatidylserin, synthetischen Phospholipiden wie DOPC: Dioleoylphosphatidylcholin, DSPC: Diastearoylphosphatidylcholin, umfasst, und ein Sterol aus der Gruppe, umfassend ein Cholesterin, Ergosterol, Sitosterol, ausgewählt ist, und davon, und die Lösungsmittel aus Ethanol, Methanol, Dichlormethan, destilliertem Wasser ausgewählt sein können.

3. Verfahren zur Herstellung der Formulierung, wie sie in Anspruch 1 beansprucht ist, umfassend die Schritte:

a. Solubilisieren von 5 mg Wurzelextrakt von neuem Chemotyp von *Withania somnifera* (NMITLI118RT+) in 1 ml Ethanol;
b. Zugeben von 4 ml Dichlormethan zu dem in Schritt a erhaltenen Gemisch;
c. Solubilisieren von Sojalecithin (S), 40 mg, und Cholesterin (C), 2 mg, in dem in Schritt (b) erhaltenen Lösungsmittelgemisch;
d. Eindampfen des in Schritt (c) erhaltenen Gehaltes an einem Rotationsverdampfer mit 140 UpM und bei 50°C für 1 Stunde zur Trockne, um einen Film zu erhalten;
e. Trocknen des in Schritt d erhaltenen Films;
f. Rehydratisieren des in Schritt (e) erhaltenen Films mit 5 ml Phosphatpuffer (pH 7,0) bei 50°C für 1 Stunde unter Erhalt einer liposomalen Dispersion;
g. Homogenisieren der in Schritt (f) erhaltenen liposomalen Dispersion unter Erhalt der Formulierung und
h. Reinigung der in Schritt (g) erhaltenen liposomalen Dispersion durch Lyophilisierung.

4. Formulierung, wie sie in Anspruch 1 beansprucht ist, die zur Wiederherstellung der Gehirnfunktion, zum Schutz vor neurologischen Störungen und Schlaganfall verwendbar ist.

5. Formulierung, wie sie in Anspruch 1-4 beansprucht ist, wobei die Konzentration des Wurzelextraktes des neuen Chemotyps von *Withania somnifera* (NMITLI118RT+) 0,1 - 5,0 mg/ml ist.

**Revendications**

1. Formulation utile pour l'administration d'un agent neuroprotecteur, qui comprend ledit agent neuroprotecteur dans un véhicule, le véhicule étant un liposome, dans laquelle ledit agent neuroprotecteur est un extrait de racine de *Withania somnifera* appelé NMITLI118RT+.

**2.** Formulation selon la revendication 1, dans laquelle le liposome comprend un lipide sélectionné dans le groupe constitué de la phosphatidylcholine (phosphatidylcholine d'œuf, lécithine de soja), des phosphoglycérides, des sphingolipides, de la phosphatidyléthanolamine, de la phosphatidylsérine, des phospholipides synthétiques comme DOPC : dioléoyl-phosphatidylcholine, DSPC : distéaroyl-phosphatidylcholine et un stérol est sélectionné dans le groupe constitué du cholestérol; de l'ergostérol; du sitostérol, et les solvants peuvent être sélectionnés parmi l'éthanol, le méthanol, le dichlorométhane, l'eau distillée.

**3.** Procédé pour préparer la formulation selon la revendication 1, comprenant les étapes suivantes :

a) solubiliser 5 mg d'extrait de racine du nouveau chimiotype de *Withania somnifera* (NMITLI118RT+) dans 1 mL d'éthanol ;
b) ajouter 4 mL de dichlorométhane au mélange obtenu à l'étape a) ;
c) solubiliser 40 mg de lécithine de soja (S) et 2 mg de cholestérol (C) dans le mélange de solvant obtenu à l'étape b) ;
d) évaporer à sec le contenu obtenu à l'étape c) sur un évaporateur rotatif à 140 tr/min et 50 °C pendant 1 heure pour obtenir un film ;
e) sécher le film obtenu à l'étape d) ;
f) réhydrater le film obtenu à l'étape e) avec 5 mL de tampon phosphate (pH 7) à 50 °C pendant 1 heure pour obtenir une dispersion liposomale ;
g) homogénéiser la dispersion liposomale obtenue à l'étape f) pour obtenir la formulation ; et
h) purifier la dispersion liposomale obtenue à l'étape g) par lyophilisation.

**4.** Formulation selon la revendication 1 utile pour la restauration de la fonction cérébrale, la protection contre les défaillances neurologiques et l'accident vasculaire cérébral.

**5.** Formulation selon les revendications 1 à 4, dans laquelle la concentration de l'extrait de racine du nouveau chimiotype de *Withania somnifera* (NMITLI118RT+) va de 0,1 à 5,0 mg/mL.

Figure 1

Figure 1 of Figures 1-16

Fig1 : Withanolide A

Figure 2

Figure 2 of Figures 1-16

**Fig 2:** Fingerprint pattern of NMITLI118RT+ (Overlay comparison of all 4 batches with standard Withanolide A)

Figure 3

Figure 3 of Figures 1-16

A

B

**Fig. 3:** HPLC-Chromatograms of (A) Fill material obtained under example 34 (B): NMITLI118RT+ used for formulating the fill material.

Figure 4                                    Figure 4 of Figures 1-
16

(A)                                          (B)

**Fig.4:** IR Spectra of (A): NMITLI118RT+; (B): NMITLI118RT+ loaded delivery
system

Figure 5
16

Figure 5 of Figures 1-

**Fig. 5:** DSC-TGA Thermograms of (A): Withanolide A (pure standard); (B): NMITLI118RT+; (C): Lipid-mixture used to prepare formulation; (D): NMITLI118RT+ loaded delivery system (F4)

Figure 6

Figure 6 of Figures 1-
16

200 nm

200 nm

A

B

Fig 6: Transmission electron microscopy images for NMITLI118RT+ loaded liposomal preprations (F4) (A): View 1; (B): View 2

Figure 7

Figure 7 of Figures 1-16

# P<0.001 vs Sham

*** P<0.001 vs MCAO

Fig 7: Effect of pre treatment (1 hr) of NMITLI118RT+ extract, Liposomal formulation and fill material of NMITLI118RT+ on MDA level following I/R injury.

Figure 8 of Figures 1-16

Figure 8

# P<0.001 vs Sham
*** P<0.001 vs MCAO

**Fig 8:** Effect of post treatment (6 hr) of NMITLI118RT+ extract, Liposomal formulation and fill material of NMITLI118RT+ on MDA level following I/R injury.

Figure 9                                                                Figure 9 of Figures 1-16

# P<0.001 vs Sham

Fig 9: Effect of pre treatment (1 hr) of NMITLI118RT+ extract, Liposomal

*** P<0.001 vs MCAO

formulation and fill material of NMITLI118RT+ on GSH level following I/R injury.

Figure 10

Figure 10 of Figures 1-16

# P<0.001 vs Sham

\*\*\* P<0.001 vs MCAO

Figure 11
16

Figure 11 of Figures 1-

# P<0.001 vs Sham

*** P<0.001 vs MCAO

Fig 11: Effect of pre treatment (1 hr) of NMITLI118RT+ extract, Liposomal formulation and fill material of NMITLI118RT+ on Neurological Deficit following I/R injury.

Figure 12　　　　　　　　　　　　　　　　　Figure 12 of Figures 1-16

# P<0.001 vs Sham

*** P<0.001 vs MCAO

**Fig 12:** Effect of post treatment (6 hr) of NMITLI118RT+ extract, Liposomal formulation and fill material of NMITLI118RT+ on Neurological Deficit following I/R injury.

Figure 13 of Figures 1-

Figure 13
16

# P<0.001 vs Sham

*** P<0.001 vs MCAO

Fig 13: Effect of pre treatment (1 hr) of NMITLI118RT+ extract, Liposomal formulation and fill material of NMITLI118RT+ on cerebral infarct following I/R injury.

Figure 14

Figure 14 of Figures 1-16

SHAM

MCAO

NMITLI 118R

LIQUID FORMULATION

CAPSULE FILL MATERIAL

**Fig 14:** Brain slices stained with TTC showing the effect of pre treatment (1 hr) of NMITLI118RT+ extract, Liposomal formulation and fill material of NMITLI118RT+ following I/R injury.

Figure 15 Figure 15 of Figures 1-16

# P<0.001 vs Sham

*** P<0.001 vs MCAO

**Fig 15:** Effect of post treatment (6 hr) of NMITLI118RT+ extract, Liposomal formulation and fill material of NMITLI118RT+ on cerebral infarct following I/R injury.

**Figure 16**

Figure 16 of Figures 1-16

SHA

MCA

NMITLI

LIQUID

CAPSULE FILL

**Fig 16:** Brain slices stained with TTC showing the effect of pre treatment (1 hr) of NMITLI 118RT+ extract, Liposomal formulation and fill material of NMITLI118RT+ following I/R injury.

**EP 3 200 770 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0103715 A1

- WO 2007014334 A2

### Non-patent literature cited in the description

- **CHAURASIYA ND ; SANGWAN RS ; MISHRA L N ; TULI R ; SANGWAN N S.** Metabolic clustering of a core collection of Indian ginseng Withania somnifera Dunal through DNA. *Isoenzyme, polypeptide and withanolide profile diversity-Fitoterapia,* 2009, vol. 80 (8), 496-505
- **DEMAERSCHALK, B.M. ; HWANG, H.M. ; LEUNG, G.** US cost burden of ischemic stroke: A systematic, literature review. *Am. J. Manag. Care,* 2010, vol. 16, 525-533
- Explaining stroke. National Stroke Association, 1-18
- **Y. K. GUPTA ; SEEMA BRIYAL ; ANIL GULATI.** Therapeutic potential of herbal drugs in cerebral ischemia. *Indian J Physiol Pharmacol,* 2010, vol. 54 (2), 99-122
- **TAKAYUKI ISHII ; TOMOHIRO ASAI ; TATSUYA FUKUTA ; DAI OYAMA ; NODOKA YASUDA ; YURIKA AGATO ; KOSUKE SHIMIZU ; TETSUO MINAMINO ; NAOTO OKU.** A Single injection of liposomal asialo-erythropoietin improves motor function deficit caused by cerebral ischemia/reperfusion. *International Journal of Pharmaceutics,* 2012, vol. 439, 269-274
- **LAPCHAK, P.A. ; SCHUBERT, D.R. ; MAHER, P.A.** Delayed treatment with a novel neurotrophic compound reduces behavioral deficits in rabbit ischemic stroke. *J. Neurochem.,* 2010, vol. 116, 122-131
- **CHAUDHARY G ; SHARMA U ; JAGANNATHAN NR ; GUPTA YK.** Evaluation of Withania somnifera in a middle cerebral artery occlusion model of stroke in rats. *Clin Exp Pharmacol Physiol.,* 2003, vol. 30, 399-404
- **CHAURASIYA ND ; SANGWAN RS ; MISRA LN ; TULI R ; SANGWAN NS.** Metabolic clustering of a core collection of Indian ginseng Withania somnifera Dunal through DNA, isoenzyme, polypeptide and withanolide profile diversity. *Fitoterapia,* December 2009, vol. 80 (8), 496-505

- **DHAR RS ; VERMA V ; SURI KA ; SANGWAN RS ; SATTI NK ; KUMAR A et al.** Phytochemical and genetic analysis in selected chemotypes of Withania somnifera. *Phytochemistry,* October 2006, vol. 67 (20), 2269-76
- **RAJASEKAR S ; ELANGO R.** Estimation of alkaloid content of Ashwagandha (Withania somnifera) with HPLC Methods. *J Exp Sci.,* 2011, vol. 2 (5), 39-41
- **MATHUR R ; GUPTA SK ; SINGH N ; MATHUR S ; KOCHUPILLAI V ; VELPANDIAN T.** Evaluation of the effect of Withania somnifera root extracts on cell cycle and angiogenesis. *J Ethnopharmacol.,* 2006, vol. 105, 336-41
- **OJHA SK ; ARYA DS.** Withania somnifera Dunal (Ashwagandha): A promising remedy for cardiovascular diseases. *World J Med Sci.,* 2009, vol. 4, 156-58
- **KUSHWAHA S ; BETSY A ; CHAWLA P.** Effect of Ashwagandha (Withania somnifera) Root Powder Supplementation in Treatment of Hypertension. *Ethno Med.,* 2012, vol. 6, 111-15
- **MISHRA LC ; SINGH BB ; DAGENAIS S.** *Scientific basis for the therapeutic use of Withania somnifera (Ashwagandha): A review,* 2000, vol. 5, 334-46
- **BHATTACHARYA SK ; BHATTACHARYA A ; SAIRAM K ; GHOSAL S.** Anxiolytic-antidepressant activity of Withania somnifera glycowithanolides: an experimental study. *Phytomedicine,* 2000, vol. 7, 463-9
- **KUSHWAHA S ; SONI VK ; SINGH PK ; BANO N ; KUMAR A ; SANGWAN RS et al.** Withania somnifera chemotypes NMITLI 101R, NMITLI 118R, NMITLI 128R and withaferin A protect Mastomys coucha from Brugia malayi infection. *Parasite Immunol.,* 2012, vol. 34, 199-209
- **CHAURASIYA ND ; SANGWAN RS ; MISRA LN ; TULI R ; SANGWAN NS.** Metabolic clustering of a core collection of Indian ginseng Withania somnifera Dunal through DNA, isoenzyme, polypeptide and withanolide profile diversity. *Fitoterapia,* 2009, vol. 80, 496-505

- **DHAR RS ; VERMA V ; SURI KA ; SANGWAN RS ; SATTI NK ; KUMAR A et al.** Phytochemical and genetic analysis in selected chemotypes of Withania somnifera. *Phytochemistry,* 2006, vol. 67, 2269-76
- **GANZERA M ; CHOUDHARY MI ; KHAN IA.** Quantitative HPLC analysis of withanolides in Withania somnifera. *Fitoterapia,* 2003, vol. 74, 68-76
- **S. KUSHWAHA ; V. K. SONI ; P. K. SINGH ; N. BANO ; A. KUMAR ; R. S. SANGWAN ; S. MISRA-BHATTACHARYA.** Withania somnifera chemotypes NMITLI 101R, NMITLI 118R, NMITLI 128R and withaferin A protect Mastomys coucha from Brugia malayi infection. *Parasite Immunology,* 2012, vol. 34, 199-209
- Reevaluation of bioactivity and antioxidant activity of Myrtus communis extract before and after encapsulation in liposomes. **OLGA GORTZI ; STAVROS LALAS ; IOANNA CHINOU ; JOHN TSAKINS.** Eur Food Res Technol. Springer-Verlag, 2007, 1-8
- **J.F. HATCHER ; K. TUREYEN.** CDP-choline liposomes provide significant reduction in infarction over free CDP-choline in stroke Rao Muralikrishna Adibhatla. *Brain Research,* 2005, vol. 1058, 193-197
- **JESUS AGULLA ; BÁRBARA ARGIBAY ; MARIA PÉREZ-MATO ; JOSE CATILLO.** Serial MRI study of the enhanced therapeutic effects of liposome-encapsulated citicoline in cerebral ischemia Pedro Ramos-Cabrer. *International Journal of Pharmaceutics,* 2011, vol. 405, 22 8-233
- **TAKAYUKI ISHII ; TOMOHIRO ASAI ; DAI OYAMA ; YURIKA AGATO ; NODOKA YASUDA ; TATSUYA FUKUTA ; KOSUKE SHIMIZU ; TETSUO MINAMINO ; NAOTO OKU.** Treatment of cerebral ischemia-reperfusion injury with PEGylated liposomes encapsulating FK506. *The FASEB Journal,* 27 April 2013, vol. 2, 1-9
- **TAKAYUKI ISHII ; TOMOHIRO ASAI ; DAI OYAMA ; TATSUYA FUKUTA ; NODOKA YASUDA ; KOSUKE SHIMIZU ; TETSUO MINAMINO ; NAOTO OKU.** *Journal of controlled release,* May 2012, vol. 160 (130), 81-87